# EUROPEAN PATENT SPECIFICATION

(11) **EP 0 531 458 B1**
(45) Date of publication and mention of the grant of the patent: **13.08.1997**
(21) Application number: 91920939.5
(22) Date of filing: 30.04.1991
(51) Int. Cl.: C07D 319/12

(54) **PROCESS FOR THE RAPID PRODUCTION OF CYCLIC ESTERS**
VERFAHREN ZUR SCHNELLEN HERSTELLUNG CYCLISCHER ESTER
PROCEDE DE PRODUCTION RAPIDE D'ESTERS CYCLIQUES

(30) Priority: 03.05.1990 US 521063; 08.05.1990 US 520348; 21.09.1990 US 586157
(43) Date of publication of application: 17.03.1993
(73) Proprietor: E.I. DU PONT DE NEMOURS AND COMPANY, Wilmington Delaware 19898 (US)
(72) Inventor: BHATIA, Kamlesh, Kumar, Newark, DE 19713 (US)
(74) Representative: Jones, Alan John
(86) International application number: US9102835
(87) International publication number: WO9117155

(56) References cited:
- US-A- 3 597 450
- US-A- 4 727 163
- US-A- 4 835 293

## Description

### FIELD OF THE INVENTION

This invention relates to an improved process for preparing cyclic esters by thermolysis of an alpha-hydroxycarboxylic acid, ester, a nitrogen based salt or an oligomer thereof at a short residence time. More particularly, the invention relates to a gas-assisted atmospheric pressure process that provides for rapid production of the cyclic esters, in particular, lactide.

### BACKGROUND OF THE INVENTION

Dimeric cyclic esters such as lactide are polymerizable to high molecular weight poly(hydroxycarboxylic) acids which are of great interest for their hydrolytic and biodegradable properties. For example, they have long been of interest for such biomedical uses as sutures and staples. More recently, they have become of interest for the manufacture of articles of commerce for non-biomedical uses that would be degradable in the environment, in particular hydrolytically, to environmentally acceptable products. For most, if not all, such uses it is preferred that the degradable polymer be made from a dimeric cyclic ester. However, dimeric cyclic esters made by existing technology are too costly for such non-medical uses because of low process yields, undesirable by-product formation and cumbersome processing.

The preparation of the dimeric cyclic esters of alpha-hydroxycarboxylic acids is an old and much studied process. Heretofore, the preparation has been conducted in two generally distinct batch steps involving first preparing an oligomer of the hydroxycarboxylic acid (i.e., a relatively short-chain condensation polymer thereof), then heating the polymer under reduced pressure to generate the desired cyclic ester. Production of cyclic esters is discussed in the following references: Gruter et al., U.S. Patent No. 1,095,205 (1914); Lowe, U.S. Patent No. 2,668,162 (1954); Bellis, U.S. Patent No. 4,727,163 (1988); Bhatia, U.S. Patent No. 4,835,293; and Muller, German Patent Application Publications Nos. 36 32 109 and 37 08 915 (1988). Such processes are not economically attractive for non-medical applications because they can require hours of reaction time at high temperatures for the conversion of the polymeric intermediate to the cyclic ester. Further, long residence times at the high temperatures employed can often result in side reactions, leading, for example, to unwanted isomers, charring of the polymer and consequently difficult to handle reactor heels.

### SUMMARY OF THE INVENTION

The present invention is based on the discovery that conversion of an alpha-hydroxycarboxylic acid or an oligomer of an alpha-hydroxycarboxylic acid moiety as defined herein, such as lactic acid, to a cyclic ester, such as lactide, proceeds more rapidly than heretofore believed, so that when there is a high interfacial area between the liquid and the gas, and the flow rate of the gas and its volume relative to the amount of oligomer are sufficiently large, i.e at a hereinafter defined minimum rate, the cyclic ester can be removed substantially as fast as it is formed. Thus, the process is capable of converting lactic acid oligomer, for example, to lactide very rapidly. This makes feasible a short residence time continuous process.

Thus, the process of the present invention offers numerous advantages over the art. It substantially reduces the time required for converting an alpha-hydroxycarboxylic based oligomeric material as defined above into the desired cyclic ester. In contrast to the prior processes, which require hours for such conversion, the subject process can produce a cyclic ester such as lactide in much less time. Consequently, the hold-up of reaction mass can be minimized so that loss of potential cyclic ester yield through racemization and charring of the reaction mass can also be minimized. The invention process is therefore capable of producing a cyclic ester such as lactide rapidly and in high yields. Through recycling of unreacted starting material (i.e., unconverted reaction mass), the conversion of cyclic ester can be increased to still higher values.

A first embodiment of the present invention relates to a continuous process for preparing a cyclic ester having the formula, 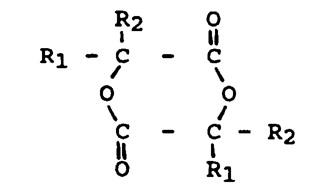 wherein R₁ and R₂ are independently a hydrogen or an aliphatic hydrocarbyl radical having 1 to 6 carbon atoms, which process comprises:
(i) continuously feeding a reactant feed stream containing at least one of (a) an alpha-hydroxycarboxylic acid, HOCR₁R₂CO₂H, and (b) a salt of (a) into the inlet end of a reactor having an inlet end and a product outlet end and an inlet for introducing a substance that is gaseous and non-reactive at the operating temperature; while
(ii) maintaining the reaction zone at a temperature and pressure effective to result in the formation of a fluid reaction mass containing cyclic ester;
(iii) continuously passing a flow of said substance that is gaseous and non-reactive at the reaction temperature through said inlet, the flow being sufficiently large to sweep cyclic ester from the reaction mass and form a gaseous product stream containing the gas and cyclic ester;
(iv) removing the product stream of (iii) from the reaction zone; and
(v) recovering the cyclic ester from the product stream.

A second embodiment of the present invention relates to an improved process for preparing a cyclic ester having the formula: 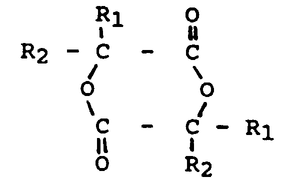 wherein R₁ and R₂ are independently a hydrogen or an aliphatic hydrocarbyl radical having 1 to 6 carbon atoms, which process comprises:
(i) feeding an oligomer of an alpha-hydroxycarboxylic acid, HOCR₁R₂CO₂H, or an ester or a nitrogen based salt thereof, into a reaction zone maintained at a temperature sufficient to depolymerize the oligomer and form cyclic ester;
(ii) continuously feeding into the reaction zone a substance that is gaseous and non-reactive at said temperature, said substance forming a gaseous stream contacting the oligomer so as to form a large interfacial area with the oligomer; said gaseous substance being fed in an amount sufficient to strip cyclic ester from the oligomer substantially as fast as it is formed, the gaseous feed being at least about 90 standard cubic feet per minute (scfm) per cubic foot of oligomer in the reaction zone;
(iii) removing the gas stream containing cyclic ester from the reaction zone; and
(iv) recovering the cyclic ester from the gas stream.

In an aspect of the present invention, the processes of the first and/or second embodiments are carried out batchwise.

In another aspect of the present invention, the feed system is liquid, and normally preheated so as to reduce the heat load on the reactor.

In one aspect of the second embodiment discussed above, the oligomer is continuously fed to the reaction zone, and the cyclic ester is continuously removed from the reaction zone.

In another more specific aspect of the present invention, the reactant feed rate and the product stream removal rate are coordinated so as to establish a steady state in that the quantity of reaction mass is maintained substantially constant within the reaction zone.

In yet another more specific continuous process of the second embodiment discussed above, the rates of oligomer feed, product stream and the removal rate of unconverted oligomer are coordinated so as to establish a steady state in that the quantity of reaction mass is maintained substantially constant within the reaction zone.

In particular, one aspect of the process of the present invention is directed to the preparation of lactide, including L-lactide and D-lactide, in high yields and high states of purity at high conversion rates, starting with lactic acid, an ester, a nitrogen based salt or an oligomer thereof.

Further, operating at pressures of about atmospheric reduces investment and operating costs by eliminating the costly equipment required for maintaining the low reduced pressures utilized heretofore in the art. The stripping at atmospheric and higher pressures eliminates a potential for an explosive atmosphere forming within the reactor that can sometimes result from air leaks, especially at reduced pressures.

### BRIEF DESCRIPTION OF THE DRAWING

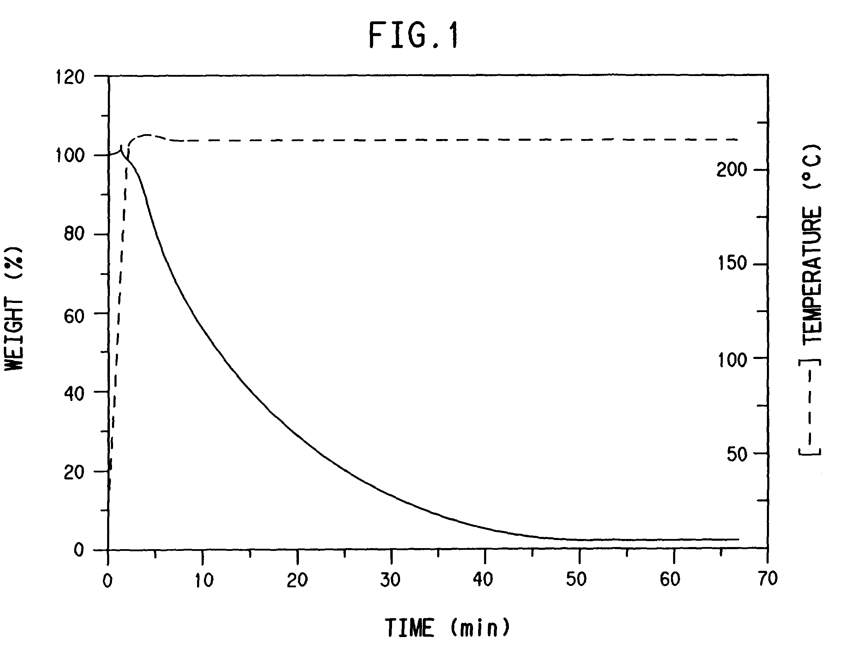

Figure 1 is a graphical representation of a thermo-gravimetric analysis (TGA) of depolymerizing an oligomer produced in accordance with Example 3.

### DETAILED DESCRIPTION OF THE INVENTION

In a first embodiment of the present invention, the process is conducted by continuously introducing an alpha-hydroxycarboxylic acid or a heat-dissociable salt (e.g., an amine salt) thereof into a reaction zone which has been preheated to a temperature effective to convert the alpha-hydroxycarboxylic acid and/or salt thereof to a cyclic ester. Substantially simultaneously, a substantially constant flow of a cyclic ester-stripping agent, as defined above, is passed into the reaction mass to bring it into intimate contact with the reaction mass and to carry the reaction products away from the reaction zone. The product stream removed from the reaction zone contains the cyclic product along with other reaction products, for example, in the case of a lactic acid feed, free water, water of reaction and unconverted lactic acid. The cyclic ester may be recovered by any of the methods known to the art, including solvent-scrubbing. One such solvent-scrubbing method is disclosed in Bhatia U.S. Patent No. 4,835,293, to which reference may be made. Unreacted alpha-hydroxycarboxylic acid recovered from the reaction product stream can be recycled to the reaction zone as well as any oligomeric purge from the reactor, either along or in conjunction with fresh starting material.

The alpha-hydroxycarboxylic based feed stream material of the first embodiment includes:
(a) alpha-hydroxycarboxylic acids, HOCR₁R₂CO₂H, where R₁ and R₂ can independently be hydrogen or a C₁-C₆ aliphatic hydrocarbyl radical;
(b) a salt, for example, an alkali metal salt or a heat-dissociable ammonium or amine salt, HOCR₁R₂CO₂HA, where A is ammonia or an amine and the salt is dissociable into the acid of item (a) and the nitrogen based salt at the operating temperatures.

For example, R₁ and R₂, when other than H in the above formulas, are C₁-C₄ alkyl groups. In some aspects, R₁ and R₂ are H or methyl, as in glycolic acid (R₁=R₂=H) and lactic acid (R₁=H, R₂=CH₃).

The amine salt of the alpha-hydroxycarboxylic acid can be any salt that is dissociable into the acid and the amine at process temperatures. For example, it will be a salt of a tertiary amine, more preferably aliphatic, such as trimethylamine, triethylamine, dimethylethylamine, tributylamine or the like.

In a second embodiment of the present invention, the process is conducted by introducing an oligomer of an alpha-hydroxycarboxylic acid, an ester thereof or a nitrogen based salt thereof into a reaction zone and heating it to a temperature effective to depolymerize it to cyclic ester. Substantially simultaneously, a substantially constant flow of a gaseous cyclic stripping agent, as defined above, is passed into the liquid reaction mass in an amount and at a rate so as to create a large interfacial area between the fluid reaction mass and the gas, and the amount of gas is sufficient to strip cyclic ester rapidly from the reaction mass. The stripping gas forms a product stream containing cyclic ester and other volatile material that may be present in the cyclic ester recovered. The cyclic ester may be recovered by any of the methods known to the art, such as solvent-scrubbing or condensation. One such solvent-scrubbing method is disclosed in Bhatia U.S. Patent No. 4,835,293, to which reference may be made. Lactic acid and its linear dimer recovered from the reaction product stream in the recovery of the cyclic ester therefrom can be recycled to the reaction zone, either alone or in conjunction with fresh starting material. A purged stream of reaction mass removed from the reactor may also be recycled. It is understood that the term "continuous feed" in the continuous process embodiment of this invention comprises both constant and pulsed feeds.

The oligomeric feed material of the second embodiment comprises a polymer of an alpha-hydroxycarboxylic acid, ester and/or a nitrogen based salt thereof, viz., 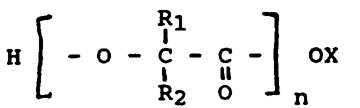 where n is an integer of 2 to 12, X is independently H, R₃ or cationic group HA, R₁, R₂ and R₃ are independently H or a C₁-C₆ hydrocarbyl radical, and A is a nitrogen base. Ordinarily, R₁, R₂ and R₃, when other than H in the above formula, is an alkyl group. For example, R₁ and R₂ are H or methyl, as in glycolic acid (R₁=R₂=H) and lactic acid (R₁=H, R₂=methyl). Further, the cationic group is derived from a nitrogen base such as ammonia or alkyl amine. Moreover, the nitrogen base is ammonia or a tertiary amine such as trimethylamine, triethylamine, methyldiethylamine, tripropylamine, tributylamine or the like. The molecular weight of the oligomer can vary widely; generally for the production of lactide it will not be greater than about 1000 for ease of handling. Moreover, the oligomeric polymer should be so constituted, in terms of n, R₁, R₂, R₃ and HA, that it can be liquefied at the operating temperature for depolymerizing it to cyclic ester. Liquidity for purposes of continuously or intermittently feeding the oligomer to the reaction zone can be achieved by preheating the material and/or adding liquidizing quantities, usually minor ones, of a compatible solvent as needed to render the composition conveniently pumpable.

The process of the present invention will ordinarily be conducted in the presence of a catalyst, carried in the feed stream along with the oligomeric reactant, or it can be incorporated directly into the reaction mass. The catalyst should be suitable for promoting (1) the condensation of the alpha-hydroxycarboxylic to an oligomer, or (2) the thermolysis of the oligomers to cyclic esters. The catalysts are generally metals or compounds of metals of groups IV, V and VIII of the Periodic Table. Examples of suitable catalysts are metals of groups IV, notably Sn as the metal (powdered), oxide, halogenide or carboxylate, or V, notably Sb, usually as the oxide Sb₂O₃. Specific examples of a suitable tin catalyst are Sn (II) carboxylates, especially those that are soluble in the feed stream and the resulting reaction mixture, exemplified by stannous bis(2-ethylhexylcarboxylate), commonly referred to as stannous octoate.

The catalyst will be employed in catalytically effective amounts, which can vary widely depending upon the particular feed material employed and reaction conditions. The optimum catalytically-effective amounts for any particular system can readily be determined through trial runs. For example, with stannous octoate as the catalyst, the quantity will generally be such that the reaction mass contains from about 0.1 to about 1.5% by weight, more usually from about 0.3 to 0.9%.

The reactor may also contain a substantially inert diluent or solvent for the oligomer, such as a high-boiling liquid heat transfer medium, to help minimize localized overheating and charring of the oligomer during its depolymerization to the cyclic ester.

The gaseous agent for entraining/carrying/ sweeping the cyclic ester and water of reaction out of the reaction mixture and out of the reactor may be any substance that is gaseous and stable at the operating temperatures and pressures and is inert to the starting material, reaction mass components and reaction products. It may be normally gaseous, such as nitrogen, argon, carbon monoxide or dioxide or low molecular weight hydrocarbon. It may be normally non-gaseous but gaseous at reaction temperature and pressure. Normally nitrogen or N₂ is utilized as a gaseous agent due to its inertness and ready availability. Ordinarily, the inert gas will be preheated to or reasonably close to the operating temperature and will be injected below the surface of the reaction mass material in the reaction zone; for example, introduced below the agitator of a stirred tank reactor or fed into the bottom of a vertically disposed reactor so that it can counter-currently contact down-flowing oligomer. This technique allows one to run a continuous reaction having a short residence time.

The flow rate of the gas should be sufficiently high so as not to limit the cyclic ester stripping rate. If the flow rate is too low, the conversion to cyclic ester may be adversely affected and its production rate limited since the gas functions importantly to carry the cyclic ester as vapor out of the reactor. While the optimum flow may vary with any particular combination of feed material, catalyst, reaction temperature and reactor design and configuration, it will normally be in the range of from about 5 to 20 moles per mole of feed, and ordinarily from 7 to 15 moles per mole of feed.

In accordance with the present invention, the inert gas flow will be at least about 90 standard cubic feet per minute (scfm) per cubic foot of oligomer (90 m³/min/m³), and ordinarily between about 180 to 600 scfm per cubic foot of oligomer (180 to 600 m³/min/m³) in the reactor. In continuous operation, under steady state conditions, it is convenient also to base the gas feed rate on the rate at which the oligomer is being fed to the reactor. On this basis, the gas flow rate will be at least 1000 scfm (and as high as 2000 to 6000 scfm) per cubic foot per minute of oligomer (1000, 2000 to 6000 m³/min/m³) being fed to the reaction zone. Suitably effective temperatures for converting oligomer to cyclic ester can vary widely, but normally will be in the range of from about 185 to 270°C, or in the range of from about 200 to 220°C. At these temperatures the residence times in the continuous embodiment would not be greater than about 45 minutes, and usually not greater than 20 minutes. The optimum temperature for any particular oligomer-to-cyclic ester conversion will vary with composition. For example, for the production of L- or D-lactide the temperature will usually range from about 190-220°C, for glycolide 220-250°C.

It will also be noted that the gaseous component helps maintain the reaction mixture in the reaction zone well-mixed, as well as to remove volatiles therefrom, and in this way helps to avoid the occurrence of localized "hot spots" (i.e., zones of unduly high temperatures), which could otherwise result in unwanted and yield-lowering degradation and charring of the reaction mass. Suitably effective temperatures for converting monomeric or alpha-hydroxycarboxylic component (i.e., an acid or ammonium or amine salt of the second embodiment) to cyclic ester, can vary widely but normally will be in the range of from about 170° to 270°C, or in the range of from about 190° to 235°C, and in the case of lactide production 195° to 220°C.

The pressure may vary from sub-atmospheric to atmospheric and super-atmospheric. Normally, it is at atmospheric pressure plus a small back pressure exerted on the product stream equipment which should be designed to keep the back pressure as low as practical, for example, less than 5 psi (34.5 KPa).

The reactor design and configuration are not critical, provided there are means (1) which permit formation and accumulation of an in situ produced fluid reaction mass, or (2) for introducing an oligomer feed, and includes means for introducing a gaseous cyclic ester-stripping agent into the reaction zone such that it directly and intimately contacts the oligomeric composition so as to give high gas-liquid interfacial area and has means for removing a gaseous stream containing cyclic ester. Thus, the reactor may be a stirred tank equipped with gas-sparging means, and usually one which admits the gas directly under the agitator. The reactor may also be a packed or sieve-plate column, or it may be of any other design known in the art for effecting intimate gas-liquid contact, for example, a bubble column, a plate column, a spray reactor or a film reactor, again with means for introducing the gaseous component such that it intimately contacts the spray or film constituting the reaction mass. Likewise, the product stream recovery and processing system may be any of those known to the art. One such reactor system is disclosed in Bhatia U.S. Patent No. 4,835,293, to which reference may be made.

In accordance with a first embodiment of the present invention, if desired alpha-hydroxycarboxylic acids can be fed to the reactor as solution (e.g., in water, acetone, etc.). Lactic acid, for example, is commercially available as concentrated aqueous solutions which may be fed directly to the reaction zone in accordance with the method of the present invention.

A reactor particularly suitable for depolymerizing oligomeric compositions in accordance with the continuous method of the second embodiment of the present invention comprises a vertically disposed columnar reactor such as a sieve plate column equipped with means for feeding liquefied oligomer at or near the top of the column, means for removing unconverted liquid reaction mass at the bottom of the column, means for feeding the gaseous component at or near the bottom of the column such that it can pass through the downcoming reaction mass in the column, an exit means at the top of the column for removing the gaseous product stream, and heating means surrounding the column for bringing the reaction mass to the desired temperature and keeping it there.

Oligomeric material to be fed to the reactor should be liquifiable at the operating temperatures. If solid or too viscous to be conveniently fed to the reactor and maintained liquid in the reaction zone, a suitable solvent such as acetone may be used in small quantities sufficient to provide a pumpable fluid mass. Usually the feed stream will be preheated to the reaction temperature selected for depolymerizing oligomer to cyclic ester.

The Examples that follow are intended to illustrate the invention, and are not to be construed as limiting it to any particular embodiment described therein.

Examples 1 and 2 were conducted in a 28 mm Oldershaw distillation column having 5 sieve plates and fitted for the present purpose with an oligomeric feed line at the top plate, a fitting for introducing the gas into the column below the bottom-most plate and a gas stream take-off line at the top of the column, a flask for collecting unconverted oligomer from the bottom of the column, and a heating mantle for temperature control. The gas stream line take-off communicated with a scrubbing system for removing cyclic ester from the gas stream. The scrubbing system was described in Bhatia, U.S. Patent No. 4,835,293.

### EXAMPLE 1

A. An oligomer of L-lactic acid was prepared by gradually heating a mixture of 754.1 g of 88% aqueous lactic acid (about 98% L-lactic acid) and 2.54 g of stannous octoate, over a 2-hour period to 167°C, while removing free water and water of condensation in a small stream of N₂ gas. 190 cc of aqueous distillate was collected during this period. The reaction mass was held at 160-176°C for another 0.5 hour, during which time another 6 cc of distillate was collected, and the rate of removal of distillate slowed to essentially nil. The reaction mass was then cooled and mixed with acetone; the resulting composition contained 78.6% oligomer and 21.4%, by weight, acetone, and was sufficiently fluid to be pumped to the reactor at room temperature.
B. The oligomer-acetone composition from A, having a temperature of just above room temperature, was continuously pumped to the top plate of the 5-plate reactor described above, at a rate of 2.2 g/minute, while a stream of N₂ gas, preheated to about 185°C, was fed at a rate of 0.14 scfm (0.004 m³/min) below the bottom-most (5th) plate. The feeds were continued for 2 hours and 15 minutes. The temperature ranged from 186 to 197°C, averaging about 190°C; the temperature at the 4th plate ranged from 205 to 212°C, most of the time at 210°C.
   During the 135 minute run, 305 g of the feed composition containing 240 g of oligomer, equivalent to a theoretical 222 g of lactide, were fed to the column and 86 g of unreacted oligomer were recovered in the collection flask, corresponding to a conversion of 64%. Reactor hold-up was essentially constant and amounted to about 11 g. The above data calculate to an inert gas rate of 2750 scfm/cubic foot/minute (2750 m³/min/m³) of the oligomer feed and 432 scfm/cubic foot (432 m³/min/m³) of oligomer hold-up in the reaction zone. The 97 g of oligomer (hold-up and bottoms) was clear and slightly yellow in color. The resident time of the oligomer in the reactor was calculated by dividing the hold-up by the oligomer flow rate. It was about 6 minutes based on the oligomeric feed rate and 17 minutes based on the bottom rate.
C. The lactide product was recovered from the N₂ gas stream exiting the column by scrubbing with acetone. The acetone solution was partially evaporated under reduced pressures to precipitate a lactide. Chilled water was added to further precipitate lactide, which was filtered, the filter cake washed on the filter with isopropyl alcohol and residual solvent removed by evaporating under reduced pressure to yield 86 g of lactide. An additional 21 g were recovered from the filtrate and washings, also by concentration under reduced pressure, filtration, washing and drying as before. The 107 g of white crystalline L-lactide corresponded to 79% of the quantity (136 g) of oligomer converted.
   This product was found to be free of the D-lactide and meso-lactide by high pressure liquid chromatography (HPLC) on a chiral column. Its purity, without recrystallization, was 99.71% by HPLC and 98.69% by differential scanning colorimetry (DSC). It showed a sharp melting point at 97°C as determined by DSC. Upon recrystallization (90% recovery), the purity improved to 99.77% by HPLA and 99.59% by DSC. The melting point was 97.5°C by DSC. The optical rotation before recrystallization was -288° and after recrystallization from isopropyl alcohol -298°, again showing a very high purity.

### EXAMPLE 2

The procedure of Example 1 was repeated except that:
(a) the oligomer itself (i.e., without added acetone), was preheated to 140°C to form a liquid feed,
(b) the oligomer feed rate was 1.5 g/minute,
(c) the temperature at the top plate ranged from 204 to 217°C, most of the time was at 215°C,
(d) the temperature at the 4th plate ranged from 189 to 196°C, most of the time was at 195°C,
(e) the inert gas rate was 3300 scfm/cubic foot/minute (3300 m³/min/m³) of oligomer feed and 432 scfm/cubic foot (432 m³/min/m³) of oligomer hold-up in the reaction zone, and
(f) the amount of unconverted oligomer was 13.8 g, corresponding to a conversion of about 93%.

The lactide product before recrystallization had a purity of 98.5% by HPLC, 99.34% by DSC, and showed a sharp melting point of 98.9°C. Its optical rotation was -294°. After recrystallization from isopropyl alcohol, the purity by DSC was 99.69%; and its optical rotation was found to be -300°. About 80% of the oligomer converted was recovered from the acetone solution as pure L-lactide product.

### EXAMPLE 3

Depolymerization of lactic acid oligomer in a stream of nitrogen was simulated using a thermo-gravimetric analysis (TGA) apparatus. The oligomer used was made from 88% lactic acid by a procedure similar to that described in Example 1. A 15.3 mg sample of the oligomer was used. The surface to volume ratio was very high: assuming the sample to be a single droplet, the interfacial area calculates to about 600 square foot per cubic foot (600 m²/m³) of the oligomer. The oligomer temperature was raised quickly in about 2 minutes to the depolymerization temperature of 215°C and held there. A relatively large stream of nitrogen (100 cc/minute) was passed through. Surprisingly, it was found that the rate of depolymerization of the oligomer was very rapid.

As seen in Figure 1, about 50% of the oligomer was converted in about 10 minutes and 70% converted in about 16-18 minutes. This demonstrates that depolymerization of the oligomers to cyclic esters can be accomplished rapidly (i.e., in a few minutes of residence time as opposed to several hours employed in the prior art), provided the reactor is designed so that it provides a large interfacial area to facilitate cyclic ester removal and there is sufficient amount of carrier gas to remove the cyclic ester from the reaction zone as rapidly as it is formed.

Examples 4 and 5 below were conducted in a stirred reactor equipped with a feed inlet, a gaseous product stream outlet and a gas sparging device for introducing N₂ gas as more fully described in Bhatia, U.S. Patent No. 4,835,293, including Fig. 1 therein.

### EXAMPLE 4

376.4 g of 88% L-lactic acid containing 2 g of stannous octoate catalyst was charged to a reactor, preheated to 217°C to reduce the heating time, while a stream of N₂ preheated to 135°C as flowing through the reactor at a rate of 0.1 scfm (0.003 m³/min) to facilitate removal of the free water present in the lactic acid and the water of condensation of lactic acid to an oligomer. Charging the preheated reactor with lactic acid dropped the temperature to 93°C, but it was quickly heated up to start removing water. Water removal was continued as the reactor temperature continued to increase. After only 20 minutes most of the water was removed, as the reactor temperature reached 178°C, lactide started to evolve and was seen freezing out from the N₂ stream in the water cooled condenser connected to the reactor.

Thereafter, lactic acid could be fed continuously with continuous generation of lactide, as described more specifically in the following Example.

### EXAMPLE 5

106.9 g of 88% L-lactic acid containing 0.25% weight stannous octoate as catalyst was charged to the reactor preheated to 215°C and water removed as in Example 4. In 19 minutes, 40.2 g of water plus the lactic acid that volatilized were collected as condensate leaving 66.7 g of oligomer in the reactor.

A continuous feed of 88% lactic acid containing 0.25% weight catalyst, preheated to 68°C, was then started and the reaction products stripped away with the N₂ stream were recovered by scrubbing it with acetone. The feed rate, N₂ rate and reactor temperature were adjusted during the next 73 minutes so as to arrive at nearly steady state operating conditions. During this period a total of 180 g of lactic acid feed was consumed and 25 g of oligomer reactor mass was drained from the reactor so that the oligomer level was about the same as when starting the continuous feed.

After the above adjustment period to achieve a nearly steady operation, the acetone solution was drained from the scrubber and fresh acetone changed to scrubber. Lactic acid containing 0.25% catalyst was fed to the reactor for the next 20 minutes. at a rate of 3 g/minute while the nitrogen heated to 164°C was sparged at a rate of 0.3 scfm (0.0085 m³/min). The reactor temperature during this period ranged between 208° and 221°C. The reaction was then stopped and the reactor contents as well as the acetone solution form the scrubber were drained.

The reaction mass, 61 g, was quite fluid and light amber in color in sharp contrast to the highly viscous and blackish reactor heels obtained in the conventional prior art processes.

The acetone solution was concentrated by vacuum stripping the acetone and then adding chilled water so as to precipitate the lactide product and retain the unconverted acid in the water. The L-lactide product was filtered, washed twice with cold water and dried. It weighed 35.6 g. The product was pure white, crystalline L-lactide and found to be 97.24% pure by differential scanning colorimetry (DSC).

The filtrate and washings from the above operation were combined and evaporated under vacuum to obtain 26 g of unconverted lactic acid.

Thus, about 55% of the lactic acid fed was converted to lactide. The recovered unconverted acid, as well as the fluid reaction mass, could be recycled to obtain a high overall yield.

Based on the 61 g of reaction mass drained from the reactor at the end of the reaction and the 3 g/minute feed rate of the acid, the reaction residence time is calculated to be about 20 minutes.

## Claims

1. An improved process for preparing a cyclic ester having the formula, 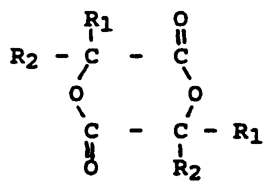 wherein R₁ and R₂ are independently a hydrogen or a C₁-C₆ aliphatic hydrocarbyl radical, which process comprises:
(i) introducing a feed material comprising alpha-hydroxycarboxylic acid, HOCR₁R₂CO₂H, or an ester or oligomer or a salt thereof, into a reaction zone maintained at a pressure and temperature effective to produce a cyclic ester;
(ii) continuously providing into the reaction zone a substance that is gaseous and non-reactive at said temperature, said substance forming a gaseous stream contacting the feed material so as to form a large interfacial area with the feed material; said gaseous substance being provided in an amount of at least 90 standard cubic feet per minute per cubic foot of oligomer (90m³/min/m³) and sufficient to strip cyclic ester from the feed material substantially as fast as it is formed;
(iii) removing the gas stream containing cyclic ester from the reaction zone; and
(iv) recovering the cyclic ester from the gas stream.

2. The process of Claim 1 wherein the feed material of (i) is continuously fed to the reaction zone, the gas feed in (ii) is at least 1000 scfm per cubic foot of oligomer being fed per minute (1000 m³/min/m³), the gas stream of (iii) is continuously removed from the reaction zone.

3. The process of Claim 2 wherein the reaction zone is maintained at a pressure of at least about atmospheric pressure.

4. The process of Claim 3 wherein the feed material comprises an oligomer and said feed material in the reaction zone contains a catalyst effective to depolymerize the oligomer to cyclic ester, the catalyst being present in a catalytically effective amount.

5. The process of Claim 4 wherein the catalyst is introduced with the oligomer fed to the reaction zone.

6. The process of Claim 1 wherein the temperature is in the range of about 180° to 270°C.

7. The process of Claim 1 wherein the feed material is preheated to a temperature at or substantially close to the reaction zone temperature.

8. The process of Claim 1 wherein the feed material is provided at a first rate, the gas stream comprising the cyclic ester is removed at a second rate and unconverted feed material is removed at a third rate, said rates being maintained substantially constant and coordinated to maintain a substantially constant quantity of feed material within the reaction zone.

9. The process of Claim 1 wherein the feed material comprises an alpha-hydroxycarboxylic acid or a salt thereof, and a catalyst effective to promote the condensation of the alpha-hydroxycarboxylic acid or a salt thereof to an oligomer and to effect conversion of the condensation product to a cyclic ester.

10. The process of Claim 1 wherein the feed material comprises at least one material selected from the group consisting of L-lactic acid, alpha-hydroxycarboxylic acid and lactic acid.

11. The process of Claim 1 wherein the cyclic ester comprises a lactide.

## Patentansprüche

1. Verbesserter Prozeß zur Herstellung eines cyclischen Esters mit der Formel 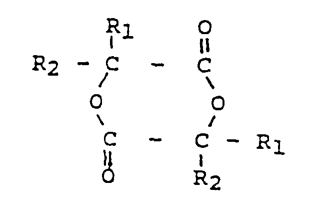 wobei R₁ und R₂ unabhängig voneinander ein Wasserstoff oder ein C₁-C₆ aliphatisches Hydrocarbyl-Radikal sind und der Prozeß folgendes umfaßt :
(i) Zufuhr eines Einsatzmaterials, das alpha-hydroxycarbonsäure, HOCR₁R₂CO₂H oder einen Ester oder ein Oligomer oder ein Salz davon enthält, in einen Reaktionsbereich, in dem ein Druck und eine Temperatur herrschen, die zur Herstellung eines cyclischen Esters geeignet sind;
(ii) kontinuierliche Zufuhr einer bei der besagten Temperatur nicht reagierenden gasförmigen Substanz in den Reaktionsbereich in der Art, daß diese Substanz einen Gasstrom bildet, der Kontakt mit dem Einsatzmaterial hat, so daß eine große Grenzfläche mit dem Einsatzmaterial entsteht; diese gasförmige Substanz muß in einer Menge von mindestens 90 Standard-Kubikfuß pro Minute pro Kubikfuß des Oligomers (90 m³/min/m³) zugeführt werden und ausreichen, um den cyclischen Ester annähernd so schnell, wie er gebildet wird, aus dem Einsatzmaterial auszutreiben;
(iii) die Herausführung des Gasstroms, der cyclischen Ester enthält, aus dem Reaktionsbereich und
(iv) die Gewinnung des cyclischen Esters aus dem Gasstrom.

2. Prozeß gemäß Anspruch 1, wobei das Einsatzmaterial von (i) kontinuierlich in den Reaktionsbereich hineingeführt wird, die Gaszufuhr von (ii) mindestens 1000 scfm pro Kubikfuß des pro Minute zugeführten Oligomers beträgt (1000 m³/min/m³) und der Gasstrom von (iii) kontinuierlich aus dem Reaktionsbereich herausgeführt wird.

3. Prozeß gemäß Anspruch 2, wobei der Reaktionsbereich unter einem Mindestdruck von annähernd atmosphärischem Druck gehalten wird.

4. Prozeß gemäß Anspruch 3, wobei das Einsatzmaterial ein Oligomer umfaßt und dieses Einsatzmaterial im Reaktionsbereich einen Katalysator enthält, der eine Depolymerisation des Oligomers zu cyclischem Ester bewirkt; der Katalysator muß dabei in einer katalytisch wirksamen Menge vorliegen.

5. Prozeß gemäß Anspruch 4, wobei der Katalysator zusammen mit dem in den Reaktionsbereich hineingeführten Oligomer zugeführt wird.

6. Prozeß gemäß Anspruch 1, wobei die Temperatur in einem Bereich von etwa 180° bis 270 °C liegt.

7. Prozeß gemäß Anspruch 1, wobei das Einsatzmaterial auf eine Temperatur vorgeheizt wird, die der Temperatur des Reaktionsbereichs entspricht oder annähernd entspricht.

8. Prozeß gemäß Anspruch 1, wobei das Einsatzmaterial mit einer ersten Geschwindigkeit zugeführt wird, der Gasstrom, der den cyclischen Ester enthält, mit einer zweiten Geschwindigkeit herausgeführt wird und nicht umgewandeltes Einsatzmaterial mit einer dritten Geschwindigkeit herausgeführt wird, wobei diese Geschwindigkeiten weitgehend konstant gehalten und so aufeinander abgestimmt werden, daß sich im Reaktionsbereich eine annähernd konstante Menge des Einsatzmaterials befindet.

9. Prozeß gemäß Anspruch 1, wobei das Einsatzmaterial eine alphahydroxycarbonsäure oder ein Salz davon umfaßt sowie einen Katalysator, der die Kondensation der alpha-hydroxycarbonsäure oder eines Salzes davon zu einem Oligomer fördert und die Umwandlung des Kondensationsprodukts in einen cyclischen Ester bewirkt.

10. Prozeß gemäß Anspruch 1, wobei das Einsatzmaterial wenigstens ein Material aus der Gruppe, die sich zusammensetzt aus L-Milchsäure, alphahydroxycarbonsäure und Milchsäure, umfaßt.

11. Prozeß gemäß Anspruch 1, wobei der cyclische Ester ein Laktid umfaßt.

## Revendications

1. Procédé amélioré pour préparer un ester cyclique répondant à la formule, 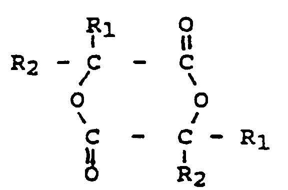 dans laquelle R₁ et R₂ représentent indépendamment un atome d'hydrogène ou un radical hydrocarbyle aliphatique en C₁-C₆, procédé qui comprend
(i) l'introduction d'un matériau d'alimentation comprenant un acide alpha-hydroxycarboxylique, HOCR₁R₂CO₂H, ou un ester ou oligomère ou un sel de celui-ci, dans une zone réactionnelle maintenue à une pression et à une température efficaces pour produire un ester cyclique;
(ii) la fourniture continue dans la zone réactionnelle d'une substance qui est gazeuse et non-réactionnelle à ladite température, ladite substance formant un courant gazeux entrant en contact avec le matériau d'alimentation de manière à former une grande surface interfaciale avec le matériau d'alimentation; ladite substance gazeuse étant fournie en une quantité d'au moins 90 pieds cubes standard par minute par pied cube d'oligomère (90m³/min/m³) et suffisante pour entraîner l'ester cyclique du matériau d'alimentation essentiellement à la vitesse à laquelle il est formé;
(iii) le retrait du courant gazeux contenant l'ester cyclique de la zone réactionnelle; et
(iv) la récupération de l'ester cyclique du courant gazeux.

2. Procédé selon la revendication 1, dans lequel le matériau d'alimentation de (i) est introduit de manière continue dans la zone réactionnelle, l'alimentation en gaz dans (ii) vaut au moins 1.000 scfm par pied cube d'oligomère introduit par minute (1.000 m³/min/m³), le courant gazeux de (iii) est retiré continuellement de la zone réactionnelle.

3. Procédé selon la revendication 2, dans lequel la zone réactionnelle est maintenue à une pression d'au moins environ la pression atmosphérique.

4. Procédé selon la revendication 3, dans lequel le matériau d'alimentation comprend un oligomère et ledit matériau d'alimentation dans la zone réactionnelle contient un catalyseur efficace pour dépolymériser l'oligomère en ester cyclique, le catalyseur étant présent en une quantité catalytiquement efficace.

5. Procédé selon la revendication 4, dans lequel le catalyseur est introduit avec l'alimentation d'oligomères dans la zone réactionnelle.

6. Procédé selon la revendication 1, dans lequel la température se situe dans la gamme d'environ 180 ° à 270 °C.

7. Procédé selon la revendication 1, dans lequel le matériau d'alimentation est préchauffé à une température égale ou essentiellement proche de la température de la zone réactionnelle.

8. Procédé selon la revendication 1, dans lequel le matériau d'alimentation est fourni à un premier taux, le courant gazeux comprenant l'ester cyclique est retiré à un deuxième taux et le matériau d'alimentation non-converti est retiré à un troisième taux, lesdits taux étant maintenus essentiellement constants et coordonnés de façon à maintenir une quantité essentiellement constante de matériau d'alimentation dans la zone réactionnelle.

9. Procédé selon la revendication 1, dans lequel le matériau d'alimentation comprend un acide alpha-hydroxycarboxylique ou un sel de celui-ci, et un catalyseur efficace pour promouvoir la condensation de l'acide alpha-hydroxycarboxylique ou d'un sel de celui-ci en un oligomère et pour effectuer la conversion du produit de condensation en un ester cyclique.

10. Procédé selon la revendication 1, dans lequel le matériau d'alimentation comprend au moins un matériau choisi parmi le groupe constitué de l'acide L-lactique, de l'acide alpha-hydroxycarboxylique et de l'acide lactique.

11. Procédé selon la revendication 1, dans lequel l'ester cyclique comprend un lactide.
